Europäisches Patentamt

European Patent Office

Office européen des brevets

(19)

(11) Numéro de publication : **0 049 182**
B1

(12) **FASCICULE DE BREVET EUROPÉEN**

(45) Date de publication du fascicule du brevet :
21.03.84

(51) Int. Cl.³ : **A 61 K 39/108**, A 61 K 37/02, A 61 K 45/05, C 12 P 21/00, C 07 G 7/00

(21) Numéro de dépôt : **81401415.5**

(22) Date de dépôt : **11.09.81**

(54) **Nouvelles glycoprotéines immunostimulantes extraites de Klebsiella Pneumoniae, leur procédé d'obtention, leur application à titre de médicaments et les compositions les renfermant.**

(30) Priorité : **19.09.80 FR 8020188**

(43) Date de publication de la demande :
**07.04.82 Bulletin 82/14**

(45) Mention de la délivrance du brevet :
**21.03.84 Bulletin 84/12**

(84) Etats contractants désignés :
**AT BE CH DE GB IT LI LU NL SE**

(56) Documents cités :
FR-A- 2 043 475
FR-A- 2 088 112
FR-A- 2 171 907
FR-A- 2 253 499
FR-A- 2 396 020
FR-A- 2 462 477

(73) Titulaire : **ROUSSEL-UCLAF**
**35, boulevard des Invalides**
**F-75007 Paris (FR)**

(72) Inventeur : **Zalisz, René**
**Résidence Pont Petit Bât. 3A 19, rue de France**
**95310-Saint-Ouen-L'Aumone (FR)**
Inventeur : **Salles, Marie-France**
**97, avenue de Villiers**
**F-75017 Paris (FR)**

(74) Mandataire : **Vieillefosse, Jean-Claude et al**
**ROUSSEL-UCLAF Boîte postale no. 9 111, route de Noisy**
**F-93230 Romainville (FR)**

Jouve, 18, rue St-Denis, 75001 Paris, France

Nouvelles glycoprotéines immunostimulantes extraites de Klebsiella Pneumoniae, leur procédé d'obtention, leur application à titre de médicaments et les compositions les renfermant

La présente invention concerne de nouvelles glycoprotéines immunostimulantes extraites de Klebsiella Pneumoniae, leur procédé d'obtention, leur application comme médicaments et les compositions les renfermant.

Un certain nombre de brevets français ont déjà décrit des glycoprotéines extraites de Klebsiella Pneumoniae, il en est ainsi, par exemple, des brevets français n° 2.043.475, 2.088.112 et 2.171.907.

La présente demande concerne des glycoprotéines plus précisément définies et a ainsi pour objet de nouvelles glycoprotéines hydrosolubles immunostimulantes extraites de Klebsiella Pneumoniae, caractérisées en ce qu'elles renferment 30 % à 45 % de protéines, 30 % à 40 % d'oses neutres, moins de 4 % d'acide glucuronique, 2 % à 5 % d'osamines et ont un poids moléculaire d'environ 350 000 daltons.

On désigne par oses neutres, notamment, des hexoses neutres tels que le glucose, le mannose ou le galactose.

Le poids moléculaire des glycoprotéines de la présente demande a été estimé par ultra centrifugation.

Les glycoprotéines de l'invention peuvent être extraites de différentes souches de Klebsiella Pneumoniae ; on retient cependant tout particulièrement celles qui proviennent de la souche déposée à l'Institut Pasteur sous le numéro 52 145.

L'étude de la structure de ces glycoprotéines à l'aide de différentes techniques chimiques, notamment, la réduction des résidus acides uroniques par le carbodiimide, la perméthylation, la dégradation uronique, l'oxydation périodique ou l'oxydation par l'oxyde de chrome, a permis de préciser leur composition et leur structure.

Les glycoprotéines selon l'invention sont formées d'une chaîne protéique sur laquelle est greffée la fraction polysaccharidique.

Les glycoprotéines préférées selon l'invention sont celles pour lesquelles la fraction protéique est composée par environ 30 % d'acides aminés acides. On entend par acides aminés acides des acides aminés tels que l'acide aspartique ou l'acide glutamique.

Les glycoprotéines préférées selon l'invention sont aussi celles pour lesquelles la fraction polysaccharidique renferme approximativement une molécule de glucose pour quatre molécules de galactose.

Parmi celles-ci, on retient tout particulièrement celles pour lesquelles la fraction polysaccharidique est essentiellement composée de la répétition de l'unité poly-saccharidique dont la structure est la suivante :

$$[(^3galactose^1)_m{-}^3galactose^1{-}^4glucose^1]_n$$

dans laquelle m est un nombre entier égal à 3, 4 ou 5. On a pu déterminer que dans cette structure, n est un nombre entier égal ou peu différent de 94 lorsque m est égal à 5.

L'invention a également pour objet un procédé d'obtention des nouvelles glycoprotéines hydrosolubles telles que définies ci-dessus, ledit procédé est caractérisé en ce que l'on traite, à l'aide d'un ammonium quaternaire, une solution de glycoprotéines obtenue par diafiltration d'un extrait de lysat de cultures de Klebsiella Pneumoniae, isole le surnageant par élimination du précipité obtenu, traite à froid à l'aide d'un alcanol de faible poids moléculaire le surnageant correspondant à une solution saline des glycoprotéines, obtient ainsi un nouveau précipité que l'on redissout dans l'eau, dialyse, lyophilise, remet en solution, filtre sur gel, puis recueille la première fraction éluée, concentre et, si désiré, amène à sec.

On peut utiliser différentes solutions de glycoprotéines au départ du procédé, ces solutions sont obtenues par diafiltration de lysats de cultures de Klebsiella Pneumoniae sur des membranes poreuses calibrées, capables de retenir les molécules d'un poids moléculaire égal ou supérieur à un poids moléculaire donné, qui est une constante pour ces membranes.

Les membranes utilisées sont, par exemple, les membranes vendues sous les noms de marque AMICON XM50, PM30 et UM2, mais on préfère utiliser des membranes dont le seuil de rétention est de 100 000 daltons, telles que les membranes commercialisées sous la désignation XM 100 ou HI P100 par la société AMICON ; les membranes tout particulièrement préférées permettent de retenir les molécules dont le poids moléculaire est supérieur à 300 000 daltons ; celles-ci sont avantageusement constituées par les membranes commercialisées sous la désignation XM300 par les sociétés AMICON et ROMICON.

La diafiltration permet de sélectionner, en solution, des molécules dont le poids moléculaire est supérieur à un poids moléculaire donné, comme on l'a dit, par le choix de la membrane, en fonction du seuil de rétention désiré. Il est évident, pour l'homme de l'art, que l'utilisation d'autres solutions de même caractéristiques, obtenues par d'autres moyens, comme, par exemple, par chromatographie sur gel polymérisé hydrophile, est tout à fait possible.

Préalablement à cette opération de sélection, le lysat peut, très avantageusement, être délipidé et débarrassé de ses acides nucléiques.

Dans des conditions de réalisation tout particulièrement préférées du procédé selon l'invention, la solution de glycoprotéines de départ est obtenue selon le procédé décrit dans le second certificat d'addition n° 2.171.907 au brevet français n° 2.043.475, c'est-à-dire par exemple par mise en culture des germes (Klebsiella pneumoniae CIP 52145 identique à la souche déposée par la demanderesse le 29 juin 1981 sous le n° I-163 à l'Institut Pasteur à Paris), lyse des germes, séchage (lyophilisation par exemple), extraction à l'aide de solvants des lipides, puis ultrafiltration et séchage, par exemple par lyophilisation. Dans ce certificat d'addition, le poids moléculaire des glycoprotéines a été estimé par la technique d'exclusion de gels.

L'ammonium quaternaire que l'on utilise pour traiter la solution de glycoprotéines de départ peut être, par exemple, le chlorure de pyridyl cétyl ammonium, mais tout particulièrement, le bromure de triméthyl cétyl ammonium ou Cétavlon $^{®}$.

Après traitement par l'ammonium quaternaire, le précipité obtenu peut être séparé du surnageant par des procédés classiques, tels que la décantation ou la filtration, mais, on préfère le séparer par centrifugation.

La solution obtenue est alors traitée à froid aux environs de + 4 °C, à l'aide d'un alcanol de faible poids moléculaire $(C_1-C_3)$ tel que le méthanol, l'éthanol, le n-propanol ou l'isopropanol.

On utilise de préférence l'éthanol.

Les résultats les plus intéressants sont obtenus par l'utilisation de six volumes d'éthanol pour un volume de solution saline, pendant une nuit à la température de + 4 °C.

Le précipité est redissous dans l'eau et dialysé pour le purifier. La dialyse est effectuée à l'aide de cellules de type classique obturées par des membranes, par exemple, de collodion ou de cellulose. On retient notamment les cellules dont la membrane est en cellulose régénérée, à diamètre moyen des pores égal à environ 2,40 nm retenant les substances dont le poids moléculaire est supérieur à 12 à 14 000 daltons. parmi les cellules à dialyse, on peut noter tout particulièrement les tubes VISKING $^{®}$.

Cette dialyse permet d'éliminer de la solution de glycoprotéines, les impuretés de faible poids moléculaire restantes, comme les traces d'alcanol.

La concentration à sec peut être effectuée par une technique classique, par exemple par atomisation ou lyophilisation.

Les lyophilisations sont effectuées de façon classique, par exemple dans des ensembles congélateur-sublimeur de taille moyenne comme les modèles SMU ou SMRG commercialisés par la Société Usifroid, des lyophilisateurs de grande taille comme, par exemple, l'ensemble formé par un congélateur CA1 et un sublimeur SMIRS tous deux commercialisés par Usifroid.

Des modèles plus petits de laboratoire peuvent aussi être utilisés, ainsi que ceux commercialisés par d'autres sociétés telles la société Sérail.

Les étapes de lyophilisation sont facultatives mais sont toutefois préférées.

La filtration sur gel peut être réalisée directement après dissolution du lyophilisat dans l'eau ou alors en utilisant la solution aqueuse de glycoprotéines. On préfère cependant tamponner la solution à filtrer et opérer l'élution avec le même tampon ; le tampon est avantageusement constitué de carbonate d'ammonium de telle manière que la solution de glycoprotéines à filtrer titre 0,1M en tampon.

Les gels utilisés peuvent être, par exemple, commercialisés sous le nom Sephadex $^{®}$ ; on préfère utiliser ceux commercialisés sous les noms Séphacryl $^{®}$ S 300 ou Ultragel $^{®}$ ACA34, particulièrement ce dernier.

La filtration peut être suivie à l'aide des procédés classiques, notamment par l'utilisation de la spectrophotométrie U.V. à 238 nm.

Dans des conditions préférentielles de mise en œuvre, le procédé ci-dessus décrit est caractérisé en ce que :

— l'ammonium quaternaire utilisé est le bromure de cétyl triméthyl ammonium ;

— l'on isole le surnageant par centrifugation ;

— l'alcanol de faible poids moléculaire est l'éthanol ;

— le gel utilisé est l'Ultragel $^{®}$ ACA34 ;

— la fraction éluée est amenée à sec par lyophilisation.

La présente demande a également pour objet les glycoprotéines telles qu'obtenues par le procédé selon l'invention.

Les glycoprotéines, objet de la présente invention, possèdent de très intéressantes propriétés pharmacologiques ; elles sont douées notamment de remarquables propriétés immunostimulantes, ainsi que d'une très bonne tolérance.

Ces propriétés sont illustrées plus loin dans la partie expérimentale.

Ces propriétés justifient l'utilisation des glycoprotéines de la présente demande, à titre de médicaments. La présente demande a ainsi également pour objet l'application, à titre de médicaments, des glycoprotéines telles que définies ci-dessus.

Parmi les médicaments de l'invention, on retient particulièrement ceux caractérisés en ce qu'ils sont constitués par les glycoprotéines telles qu'obtenues par le procédé de l'invention.

Ces médicaments trouvent, par exemple, leur emploi dans le traitement ou la prévention, chez l'homme et l'animal, des maladies infectieuses causées par les bactéries ou les virus, dans le traitement des maladies à parasites, des toxi-infections, dans le traitement des infections posthospitalières et postchirurgicales.

La dose usuelle, variable selon le produit utilisé, le sujet traité et l'affection en cause, peut être, par exemple. chez l'homme, de 1 mg à 15 mg par jour, par voie orale, de 1 à 15 mg par jour, par voie rectale et de 0,25 à 5 mg par jour, par voie parentérale.

L'invention a enfin pour objet les compositions pharmaceutiques qui renferment les glycoprotéines de la présente demande à titre de principe actif.

A titre de médicaments, les glycoprotéines de la présente demande peuvent être administrées par les voies digestive, parentérale ou locale.

Les compositions pharmaceutiques correspondantes peuvent être, par exemple, solides ou liquides et se présenter sous les formes pharmaceutiques couramment utilisées en médecine humaine, comme, par exemple, les comprimés, simples ou dragéifiés, les gélules, les granulés, les solutions, les sirops, les suppositoires, les préparations injectables lyophilisées ou non, les ovules, les crèmes, les pommades, les lotions, les gouttes, les collyres, les aérosols ; elles sont préparées selon les méthodes usuelles. Le ou les principes actifs peuvent y être incorporés à des excipients habituellement employés dans ces compositions pharmaceutiques, telles que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif, des exemples de mise en œuvre de l'invention.

## Exemple 1

20 g de produit tel qu'obtenu à l'exemple 1 du brevet français n° 2.171.907 sont dissous dans deux litres d'eau permutée.

On ajoute, lentement, environ 1,6 litre de Cetavlon à 3 %, l'ensemble est agité pendant une heure, puis centrifugé à 10 000 t/mn pendant 15 minutes. On élimine le précipité, ajoute ensuite au surnageant isolé, 3 litres d'éthanol à 95° en 15 minutes. Après une heure d'agitation, on centrifuge à 10 000 t/mn pendant 15 minutes, le surnageant ainsi obtenu est éliminé et le précipité est redissout dans 1 litre d'eau puis dialysé pendant 48 heures dans des tubes Visking® contre de l'eau permutée à + 4 °C. Au terme de cette dialyse, la solution est lyophilisée. On obtient 6,2 g de glycoprotéines dont on dissout 1 g dans 10 ml de carbonate d'ammonium 0, 1M. La solution est passée sur une colonne de 2,5 cm de diamètre renfermant 1 litre d'Ultragel® ACA34 (éluant : solution de carbonate d'ammonium 0, 1M), les fractions correspondant au premier pic d'élution (détection aux U.V. à 280 nm) sont rassemblées et lyophilisées et on obtient 0,51 g de la protéine cherchée, purifiée.

## Exemple 2

20 g de produit tel qu'obtenu à l'exemple 1 du brevet français n° 2.171.907, sont dissous dans 1 litre d'eau permutée. On ajoute sous agitation 0,800 litre de Cétavlon à 3 %. Après une heure d'agitation, on centrifuge à 10 000 t/mn, pendant 15 minutes. On élimine le précipité, ajoute au surnageant isolé 1,5 litre d'éthanol à 95°. Après une heure d'agitation, on centrifuge pendant 15 minutes à 10 000 t/mn. Le surnageant est éliminé et le précipité repris dans 0,500 litre d'eau et dialysé pendant 48 heures dans des tubes Visking® contre de l'eau permutée à + 4 °C.

Au terme de cette dialyse, on lyophilise la solution et on obtient 6,5 g des glycoprotéines cherchées que l'on purifie par passage sur Ultragel® ACA34 selon la technique décrite à l'exemple 1 et obtient 3,28 g des glycoprotéines cherchées.

## Exemple 3

On a préparé des comprimés répondant à la formule :

— glycoprotéines obtenues à l'exemple 1                                    5 mg
— excipient q.s. pour un comprimé terminé à                              100 mg
(Détail de l'excipient : lactose, amidon, talc, stéarate de magnésium).

## Exemple 4

On a préparé une pommade répondant à la formule :

— glycoprotéines obtenues à l'exemple 2                                  200 mg
— excipient q.s.p.                                                       100  g

## Etude pharmacologique

### A) Activité immunostimulante et mitogénique

On administre par voie intraplantaire à des lots de 10 souris, 40 μg du produit à tester et 40 μg de sérum albumine bovine par animal. On leur administre dix jours plus tard par voie intraveineuse, une dose

**0 049 182**

non létale et non choquante de sérum-albumine (100 µg par animal). Des animaux témoins ne reçoivent que la sérum-albumine lors de la première injection.

On mesure l'activité immunostimulante par l'augmentation de la réponse de choc anaphylactique à la sérum-albumine et l'activité mitogénique par l'augmentation du poids des ganglions drainant le point d'injection.

### 1) Activité immunostimulante

On relève le nombre d'animaux présentant un état de choc (dyspnée avec cyanose du museau allant jusqu'à la paralysie du train arrière, les convulsions et la mort), ainsi que la mortalité deux heures après l'injection intraveineuse de sérum-albumine.

Résultats

| Produit de l'exemple | % d'animaux choqués | % de morts |
|---|---|---|
| 1 | 60 | 40 |
| 2 | 100 | 50 |
| témoins | 0 | 0 |

### 2) Activité mitogénique

On sacrifie deux heures après l'injection intraveineuse de sérum albumine les survivants, prélève les ganglions poplités drainant la patte où a eu lieu l'injection, et les pèse.

L'activité mitogénique est exprimée à l'aide d'un index correspondant au rapport du poids moyen des ganglions des animaux traités avec le produit à tester, et de ceux des animaux témoins n'ayant reçu que la sérum-albumine.

Résultats

| Produit de l'exemple | Index |
|---|---|
| 1 | 9,1 |
| 2 | 7,2 |

Les produits des exemples 1 et 2 présentent donc de très bonnes activités immunostimulante et mitogénique.

### B) Stimulation des défenses non spécifiques :

Cette stimulation a été étudiée sur le test de la « Clearance » au carbone chez la souris en s'inspirant de la technique mise au point par HALPERN, qui consiste à injecter à l'animal dans le sinus oculaire, une suspension de carbone colloïdal et à apprécier en fonction du temps, la cinétique de la disparition du carbone dans le sang, en effectuant des mesures de densité optique.

Les produits sont administrés à l'animal par voie intrapéritonéale, vingt-quatre et quarante-huit heures avant le test. Les résultats sont exprimés en pourcentage d'élimination des particules de carbone par rapport aux témoins ayant reçu uniquement l'injection de carbone colloïdal et correspondant aux 100 % du nombre de particules de carbone.

| Produit de l'exemple | Doses | % d'activité par rapport aux témoins | |
|---|---|---|---|
| | | 8 minutes après l'injection | 30 minutes après l'injection |
| 1 | 0,25 mg/kg | 50 % | 70 % |
| 2 | 0,25 mg/kg | 50 % | 70 % |

L'examen de ces résultats nous permet de noter l'intense stimulation provoquée par ces deux produits sur les défenses de l'organisme.

5

C) Toxicité aiguë

La dose létale 50 (DL 50) par voie intrapéritonéale, chez la souris a été déterminée par la méthode de BEHRENS et KARBER.

Elle est de 30 mg/kg pour les glycoprotéines des exemples 1 et 2.

D) Tolérance

L'injection sous cutanée de 0,2 ml de glycoprotéines des exemples 1 et 2 à la dose de 1 000 γ/kg chez la souris, ne provoque aucune intolérance locale ou générale.

La souche déposée à l'Institut Pasteur sous le n° 52 145 a été redéposée à l'Institut Pasteur le 25 juin 1981 sous le n° I-163.

**Revendications** (pour les Etats contractants : BE, CH, DE, GB, IT, LI, LU, NL, SE)

1. Nouvelles glycoprotéines hydrosolubles immunostimulantes extraites de Klebsiella Pneumoniae, caractérisées en ce qu'elles renferment 30 % à 45 % de protéines, 30 % à 40 % d'oses neutres, moins de 4 % d'acide glucuronique, 2 % à 5 % d'osamines et ont un poids moléculaire d'environ 350 000 daltons.

2. Nouvelles glycoprotéines selon la revendication 1, caractérisées en ce qu'elles sont extraites de la souche déposée à l'Institut Pasteur sous le n° 52 145.

3. Nouvelles glycoprotéines selon la revendication 1 ou 2, caractérisées en ce que la fraction protéique est composée par environ 30 % d'acides aminés acides.

4. Nouvelles glycoprotéines selon l'une quelconque des revendications 1 à 3, caractérisées en ce que la fraction polysaccharidique renferme approximativement une molécule de glucose pour quatre molécules de galactose.

5. Nouvelles glycoprotéines selon la revendication 4, caractérisées en ce que la fraction polysaccharidique est essentiellement composée de la répétition de l'unité polysaccharidique dont la structure est la suivante :

$$-[(^3galactose^1)_m-^3galactose^1-^4glucose^1]_n-$$

dans laquelle m est un nombre entier égal à 3, 4 ou 5.

6. Procédé d'obtention de nouvelles glycoprotéines telles que définies à l'une quelconque des revendications 1 à 5, dans lequel l'on prépare un extrait de glycoprotéines tel que celui obtenu par mise en culture de germes de Klebsiella pneumoniae, lyse des germes, séchage, délipidation par extraction à l'aide de solvants des lipides, et ultrafiltration, ledit procédé étant caractérisé en ce que l'on traite la solution aqueuse de glycoprotéines ainsi obtenue à l'aide d'un sel d'ammonium quaternaire, obtient ainsi un précipité que l'on élimine, traite à froid le surnageant à l'aide d'un alcanol de faible poids moléculaire, $(C_1-C_3)$, obtient ainsi un nouveau précipité que l'on isole, redissout dans l'eau, dialyse, lyophilise, remet en solution, filtre sur gel, recueille la première fraction éluée, concentre et, si désiré, amène à sec.

7. Procédé selon la revendication 6, caractérisé en ce que
— l'ammonium quaternaire utilisé est le bromure de cétyl triméthyl ammonium ;
— l'on isole par centrifugation, le surnageant ;
— l'alcanol de faible poids moléculaire est l'éthanol ;
— le gel utilisé est l'Ultragel® ACA34 ;
— la fraction éluée est amenée à sec par lyophilisation.

8. Les nouvelles glycoprotéines telles qu'obtenues par le procédé selon la revendication 6 ou 7.

9. Médicaments, caractérisés en ce qu'ils sont constitués par les nouvelles glycoprotéines hydrosolubles telles que définies à la revendication 1 ou 2.

10. Médicaments, caractérisés en ce qu'ils sont constitués par les nouvelles glycoprotéines hydrosolubles telles que définies à l'une quelconque des revendications 3 à 5.

11. Médicaments, caractérisés en ce qu'ils sont constitués par les glycoprotéines telles que définies à la revendication 8.

12. Compositions pharmaceutiques, caractérisées en ce qu'elles renferment, à titre de principe actif, l'un au moins des médicaments tels que définis à l'une des revendications 9 ou 10.

13. Compositions pharmaceutiques, caractérisées en ce qu'elles renferment, à titre de principe actif, l'un au moins des médicaments tels que définis à la revendication 11.

**Revendications** (Pour l'Etat contractant AT)

1. Procédé d'obtention de nouvelles glycoprotéines hydrosolubles immunostimulantes extraites de Klebsiella pneumoniae, renfermant 30 % à 45 % de protéines, 30 % à 40 % d'oses neutres, moins de 4 % d'acide glucuronique, 2 % à 5 % d'osamines et ayant un poids moléculaire d'environ 350 000 daltons, procédé dans lequel l'on prépare un extrait de glycoprotéines tel que celui obtenu par mise en culture de germes de Klebsiella pneumoniae, lyse des germes, séchage, délipidation par extraction à l'aide de solvants des lipides, et ultrafiltration, ledit procédé étant caractérisé en ce que l'on traite la solution aqueuse de glycoprotéines ainsi obtenue à l'aide d'un sel d'ammonium quaternaire, obtient ainsi un précipité que l'on élimine, traite à froid le surnageant à l'aide d'un alcanol de faible poids moléculaire ($C_1$-$C_3$) obtient ainsi un nouveau précipité que l'on isole, redissout dans l'eau, dialyse, lyophilise, remet en solution, filtre sur gel, recueille la première fraction éluée, concentre et, si désiré, amène à sec.

2. Procédé selon la revendication 1, caractérisé en ce que
— l'ammonium quaternaire utilisé est le bromure de cétyl triméthyl ammonium ;
— l'on isole par centrifugation le surnageant ;
— l'alcanol de faible poids moléculaire est l'éthanol,
— le gel utilisé est l'Ultragel$^{R}$ ACA34 ;
— la fraction éluée est amenée à sec par lyophilisation.

3. Procédé selon la revendication 1 ou 2, caractérisé en ce que les glycoprotéines sont extraites de la souche déposée à l'Institut PASTEUR sous le n° 52 145.

4. Procédé selon l'une quelconque des revendications 1 à 3, caractérisé en ce que la fraction protéique est composée par environ 30 % d'acides aminés acides.

5. Procédé selon l'une quelconque des revendications 1 à 4, caractérisé en ce que la fraction polysaccharidique renferme approximativement une molécule de glucose pour quatre molécules de galactose.

6. Procédé selon la revendication 5, caractérisé en ce que la fraction polysaccharidique est essentiellement composée de la répétition de l'unité pentasaccharidique dont la structure est la suivante :

$$-[(^3galactose^1)_m-{}^3galactose^1-{}^4glucose^1]_n-$$

dans laquelle m est un nombre entier égal à 3, 4 ou 5.

**Claims** (for the Contracting States : BE, CH, DE, GB, IT, LI, LU, NL, SE)

1. New water-soluble, immuno-stimulating, glycoproteins extracted from Klebsiella Pneumoniae, characterized in that they contain 30 %-45 % of proteins, 30 %-40 % of neutral oses, less than 4 % of glucuronic acid, 2 %-5 % of osamines and have a molecular weight of about 350.000 daltons.

2. New glycoproteins according to Claim 1, characterized in that they are extracts of the strain filed at the Pasteur Institute under No. 52 145.

3. New glycoproteins according to Claim 1 or 2, characterized in that the protein fraction is made up of about 30 % of amino acids.

4. New glycoproteins according to any one of the Claims 1-3, characterized in that the polysaccharide fraction has approx. one glucose molecule per four galactose molecules.

5. New glycoproteins according to Claim 4, characterized in that the polysaccharide fraction is essentially made up of a repetition of the polysaccharide unit with the following structure :

$$-[(^3galactose^1)_m-{}^3galactose^1-{}^4glucose^1]_n-$$

in which m is an integer 3, 4 or 5.

6. Process for obtaining new glycoproteins as defined in any one of the Claims 1-5, in which a glycoprotein extract is prepared such as that obtained by culture of Klebsiella pneumoniae germs, lysis of the germs, drying, delipidation by extraction with lipide solvents and ultra filtration, the said process being characterized in that the aqueous solution of glycoproteins so obtained is treated with a quaternary ammonium salt, the resulting precipitate being eliminated, the supernatant is treated coid with an alkanol of low molecular weight ($C_1$-$C_3$), yielding a new precipitate which is isolated, redissolved in water, dialyzed, lyophilized, returned to solution, filtered on gel, and the fraction first eluted is collected, concentrated and, if desired, taken to dryness.

7. Process according to Claim 6, characterized in that :
— the quaternary ammonium utilized is cetyltrimethylammonium bromide ;
— the supernatant is isolated by centrifuging ;
— the alkanol of low molecular weight is ethanol ;
— the gel utilized is Ultragel$^{R}$ ACA34 ;
— the eluted fraction is taken to dryness by lyophilization.

8. New glycoproteins as obtained in the process according to Claim 6 or 7.

9. Medicaments, characterized in that they are made up of the new water-soluble glycoproteins as defined in Claim 1 or 2.

10. Medicaments, characterized in that they are made up of new water-soluble glycoproteins as defined in any one of the Claims 3-5.

11. Medicaments, characterized in that they are made up of glycoproteins as defined in Claim 8.

12. Pharmaceutical compositions, characterized in that they contain as active principle, one at least of the medicaments as defined in one of the Claims 9 or 10.

13. Pharmaceutical compositions, characterized in that they contain, as active principle, one at least of the medicaments as defined in Claim 11.


**Claims** (for the Contracting State AT)

1. Process for obtaining new water-soluble immunostimulating glycoproteins extracted from Klebsiella pneumonia, containing 30 %-45 % of proteins, 30 %-40 % of neutral oses, less than 4 % of glucuronic acid, 2 %-5 % of osamines and having a molecular weight of about 350.000 daltons, in which process a glycoprotein extract is prepared such as that obtained by culture of Klebsiella pneumoniae germs, lysis of the germs, drying, delipidation by extraction with lipide solvents and ultra filtration, the said process being characterized in that the aqueous solution of glycoproteins so obtained is treated with a quaternary ammonium salt, the resulting precipitate being eliminated, the supernatant is treated cold with an alkanol of low molecular weight ($C_1$-$C_3$), yielding a new precipitate which is isolated, redissolved in water, dialyzed, lyophilized, returned to solution, filtered on gel, and the fraction first eluted is collected, concentrated and, if desired, taken to dryness.

2. Process according to Claim 1, characterized in that :
— the quaternary ammonium utilized in cetyltrimethyl-ammonium bromide ;
— the supernatant is isolated by centrifuging ;
— the alkanol of low molecular weight is ethanol ;
— the gel utilized is Ultragel [R] ACA34 ;
— the eluted fraction is taken to dryness by lyophilization.

3. Process according to Claim 1 or 2, characterized in that the glycoproteins are extracts of the strain filed at the Pasteur Institute under No. 52 145.

4. Process according to any one of the Claims 1-3, characterized in that the protein fraction is made up of about 30 % of amino acids.

5. Process according to any one of the Claims 1-4, characterized in that the polysaccharide fraction contains approximately one glucose molecule per four galactose molecules.

6. Process according to Claim 5, characterized in that the polysaccharide fraction is essentially made up of a repetition of the pentasaccharide unit with the following structure :

$$—[(^3galactose^1)_m—^3galactose^1—^4glucose^1]_n—$$

in which m is an integer 3, 4 or 5.


**Ansprüche** (für die Vertragsstaaten : BE, CH, DE, GB, IT, LI, LU, NL, SE)

1. Neue, immunostimulierende, wasserlösliche, aus Klebsiella pneumoniae extrahierte Glycoproteine, dadurch gekennzeichnet, daß sie 30 bis 45 % Proteine, 30 bis 40 % neutrale Osen, weniger als 4 % Glucuronsäure und 2 bis 5 % Osamine enthalten und ein Molekulargewicht von etwa 350 000 Dalton besitzen.

2. Neue Glycoproteine gemäß Anspruch 1, dadurch gekennzeichnet, daß sie aus dem in dem Pasteur-Institut unter der Nr. 52 145 hinterlegten Stamm extrahiert werden.

3. Neue Glycoproteine gemäß Anspruch 1 oder 2, dadurch gekennzeichnet, daß die Proteinfraktion aus etwa 30 % sauren Aminosäuren besteht.

4. Neue Glycoproteine gemäß einem der Ansprüche 1 bis 3, dadurch gekennzeichnet, daß die Polysaccharidfraktion annähernd 1 Molekül Glucose je 4 Moleküle Galactose aufweist.

5. Neue Glycoproteine gemäß Anspruch 4, dadurch gekennzeichnet, daß die Polysaccharidfraktion im wesentlichen aus der Wiederholung der Polysaccharideinheit mit der folgenden Struktur

$$—[(^3Galactose^1)_m—^3Galactose^1—^4Glucose^1]_n—$$

besteht, worin m eine ganze Zahl von 3, 4 oder 5 darstellt.

6. Verfahren zur Herstellung neuer Glycoproteine gemäß einem der Ansprüche 1 bis 5, bei dem man einen Glycoproteine-Extrakt herstellt, wie denjenigen, erhalten durch Kultivierung von Stämmen von Klebsiella pneumoniae, Lyse der Stämme, Trocknung, Entfernung der Lipide durch Extraktion mit Lösungsmitteln für Lipide und Ultrafiltration, dadurch gekennzeichnet, daß man die so erhaltene, wäßrige Glycoproteine-Lösung mit einem quaternären Ammoniumsalz behandelt, so einen Niederschlag erhält, den man entfernt, die überstehende Flüssigkeit in der Kälte mit einem Alkanol mit niedrigem Molekulargewicht ($C_{1-3}$) behandelt, so einen neuen Niederschlag erhält, den man isoliert, erneut in Wasser löst, dialysiert, lyophilisiert, wiederum in Lösung bringt, einer Gelfiltration unterzieht, die erste eluierte Fraktion gewinnt, einengt und gewünschtenfalls zur Trockene bringt.

7. Verfahren gemäß Anspruch 6, dadurch gekennzeichnet, daß
— das verwendete quaternäre Ammonium Cetyltrimethyl-ammoniumbromid ist ;
— man die überstehende Flüssigkeit durch Zentrifugieren isoliert ;
— das Alkanol mit niedrigem Molekulargewicht Ethanol ist ;
— das verwendete Gel Ultragel® ACA34 ist ;
— die eluierte Fraktion durch Lyophilisierung zur Trockene gebracht wird.

8. Neue Glycoproteine, erhalten nach dem Verfahren gemäß Anspruch 6 oder 7.

9. Arzneimittel, dadurch gekennzeichnet, daß sie aus den neuen, wasserlöslichen Glycoproteinen gemäß Anspruch 1 oder 2 bestehen.

10. Arzneimittel, dadurch gekennzeichnet, daß sie aus den neuen, wasserlöslichen Glycoproteinen gemäß einem der Ansprüche 3 bis 5 bestehen.

11. Arzneimittel, dadurch gekennzeichnet, daß sie aus den Glycoproteinen gemäß Anspruch 8 bestehen.

12. Pharmazeutische Zusammensetzungen, dadurch gekennzeichnet, daß sie als Wirkstoff zumindest eines der Arzneimittel gemäß einem der Ansprüche 9 oder 10 enthalten.

13. Pharmazeutische Zusammensetzungen, dadurch gekennzeichnet, daß sie als Wirkstoff zumindest eines der Arzneimittel gemäß Anspruch 11 enthalten.

**Ansprüche** (für den Vertragsstaat AT)

1. Verfahren zur Herstellung neuer, immunostimulierender, aus Klebsiella pneumoniae extrahierter, wasserlöslicher Glycoproteine, enthaltend 30 bis 45 % Proteine, 30 bis 40 % neutrale Osen, weniger als 4 % Glucuronsäure und 2 bis 5 % Osamine, mit einem Molekulargewicht von etwa 350 000 Dalton, bei dem man einen Extrakt von glycoproteinen herstellt, wie denjenigen, erhalten durch Kultivierung von Stämmen von Klebsiella pneumoniae, Lyse der Stämme, Trocknung, Entfernung der Lipide durch Extraktion mit einem Lösungsmittel für Lipide und Ultrafiltration, dadurch gekennzeichnet, daß man die so erhaltene, wäßrige Lösung der glycoproteine mit einem quaternären Ammoniumsalz behandelt, auf diese Weise einen Niederschlag erhält, den man eliminiert, in der Kälte die überstehende Flüssigkeit mit einem Alkanol mit niedrigem Molekulargewicht ($C_{1-3}$) behandelt, so einen neuen Niederschlag erhält, den man isoliert, wieder in Wasser löst, dialysiert, lyophilisiert, erneut in Lösung bringt, einer Gelfiltration unterzieht, die erste eluierte Fraktion gewinnt, einengt und gewünschtenfalls zur Trockene bringt.

2. Verfahren gemäß Anspruch 1, dadurch gekennzeichnet, daß
— das verwendete quaternäre Ammonium Cetyltrimethylammoniumbromid ist ;
— man die überstehende Flüssigkeit durch Zentrifugation isoliert ;
— das Alkanol mit niedrigem Molekulargewicht Ethanol ist ;
— das verwendete Gel Ultragel® ACA34 ist ;
— die eluierte Fraktion durch Lyophilisierung zur Trockene gebracht wird.

3. Verfahren gemäß Anspruch 1 oder 2, dadurch gekennzeichnet, daß die glycoproteine aus dem in dem Pasteur-Institut mit der Nr. 52 145 hinterlegten Stamm extrahiert werden.

4. Verfahren gemäß einem der Ansprüche 1 bis 3, dadurch gekennzeichnet, daß die Proteinfraktion aus etwa 30 % sauren Aminosäuren besteht.

5. Verfahren gemäß einem der Ansprüche 1 bis 4, dadurch gekennzeichnet, daß die Polysaccharidfraktion annähernd 1 Molekül Glucose je 4 Moleküle Galactose enthält.

6. Verfahren gemäß Anspruch 5, dadurch gekennzeichnet, daß die Polysaccharidfraktion im wesentlichen aus einer Wiederholung der Polysaccharideinheit mit der folgenden Struktur besteht

$$-[(^3Galactose^1)_m-{}^3Galactose^1-{}^4Glucose^1]_n-$$

worin m eine ganze Zahl entsprechend 3, 4 oder 5 ist.